# EUROPEAN PATENT APPLICATION

(11) **EP 4 524 227 A1**
(43) Date of publication of application: **19.03.2025**
(21) Application number: 22941668.0
(22) Date of filing: 12.05.2022
(51) Int. Cl.: C12M 1/00, C12Q 1/6816, C12Q 1/6844

(54) **MEASURING DEVICE AND MEASURING METHOD**

(71) Applicant: HITACHI HIGH-TECH CORPORATION, Tokyo 105-6409 (JP)
(72) Inventor: KAMURA Yoshio, Tokyo 100-8280 (JP); TANAKA Junko, Tokyo 100-8280 (JP); ISHIDA Takeshi, Tokyo 100-8280 (JP)
(74) Representative: MERH-IP Matias Erny Reichl Hoffmann Patentanwälte PartG mbB
(86) International application number: PCT/JP2022/020026
(87) International publication number: WO 2023/218588

(57) **Abstract**

A measuring device includes a substrate including a plurality of through-holes for introducing and dividing a nucleic acid solution, an oil covering a first surface of the substrate and a second surface on an opposite side from the first surface of the substrate to close the through-hole, a heat conduction plate provided on the second surface side for heating the substrate, and a reflection-suppressing mechanism that suppresses reflection of excitation light emitted from the first surface side.

## Description

### Technical Field

The present invention relates to a measuring device and a measuring method, and particularly to digital PCR.

### Background Art

Conventionally, PCR or real-time PCR has been used for genetic testing. These technologies have a problem that measurement accuracy is low when a measurement target (nucleic acid) is in a trace amount. To solve this problem, digital PCR technology has recently attracted attention.

In digital PCR, a sample containing DNA to be detected is divided into a large number of minute regions, and PCR is performed on each minute region. The type of DNA present in each minute region is identified by performing discrimination between the segment containing the DNA to be detected and the segment not containing the DNA based on fluorescence intensity.

PTL 1 discloses, as a DNA detection method using digital PCR, a DNA detection method for measuring a melting temperature between DNA and a fluorescence-labeled probe that hybridizes to DNA in a droplet containing the DNA and the fluorescence-labeled probe.

### Citation List

### Patent Literature

PTL 1: JP 2018-108063 A

### Summary of Invention

### Technical Problem

In digital PCR in which melting curve analysis is combined, there are a plurality of types of genes to be measured, and examples thereof include wild-type and mutant-type genes. Since the melting curve differs depending on the type of the target gene and the fluorescence-labeled probe, it is possible to identify the type of the target gene by measuring the melting curve. In the case of simultaneously measuring a large number of genes, it is necessary to measure fluorescence intensity and a melting curve with high accuracy to reduce measurement variations of each gene.

One of the factors that lower the measurement accuracy of the fluorescence intensity and the melting curve is the presence of air bubbles. When air bubbles are generated in the vicinity of the minute region when the temperature rises, the fluorescence intensity cannot be accurately measured. Therefore, the measurement accuracy of the melting curve and the identification accuracy of the gene type deteriorate.

Since the problem of the air bubbles is different from the electrical noise, it is difficult to remove the air bubbles with signal processing or the like.

An object of the present invention is to provide a measuring device capable of suppressing unevenness of a fluorescence image and fluctuation of fluorescence intensity caused by an influence of air bubbles in melting curve analysis of a target gene. The foregoing and other objects and novel features of the present invention will become apparent from the description of the present specification and the accompanying drawings.

### Solution to Problem

An example of a measuring device according to the present invention includes:
a substrate including a plurality of through-holes for introducing and dividing a nucleic acid solution;
an oil covering a first surface of the substrate and a second surface on an opposite side from the first surface of the substrate to close the through-hole;
a heat conduction plate provided on the second surface side for heating the substrate; and
a reflection-suppressing mechanism configured to suppress reflection of excitation light emitted from the first surface side.

A measuring method according to the present invention is a measuring method using a measuring device, the measuring device including:
a substrate including a plurality of through-holes;
a heat conduction plate provided to change a temperature of the substrate; and
a reflection-suppressing mechanism configured to suppress reflection of excitation light emitted from a first surface side of the substrate on an opposite side from the heat conduction plate side,
the measuring method including:
   a step of introducing a nucleic acid solution into the substrate and fractionating the nucleic acid solution;
   a step of introducing an oil that covers a first surface of the substrate and a second surface on an opposite side from the first surface of the substrate to close the through-holes into which the nucleic acid solution is introduced;
   a step of changing the temperature of the substrate with the heat conduction plate; and
   a step of emitting excitation light from the first surface side of the substrate while changing the temperature, detecting fluorescence from the first surface side, and measuring the nucleic acid solution.

### Advantageous Effects of Invention

The present invention can provide a measuring device and a measuring method capable of suppressing the influence of air bubbles, measuring the fluorescence intensity and the melting curve with high accuracy, and identifying the type of gene with high accuracy.

### Brief Description of Drawings

FIG. 1 is a view for describing strength measured when air bubbles are generated in a lower portion of a through-hole well in a measuring device in a conventional example.
FIG. 2 is a reflection image for describing that reflected light from a heat conduction plate in a lower portion of a measuring device is observed through a through-hole well in a conventional example.
FIG. 3 is a diagram for describing that performing blackening surface treatment on a heat conduction plate in a lower portion of a measuring device causes the measurement intensity not to be affected by air bubbles in Example 1 of the present invention.
FIG. 4 is a reflection image for describing that performing blackening treatment on a heat conduction plate in a lower portion of a measuring device causes reflected light from the heat conduction plate not to be observed through a through-hole well in Example 1 of the present invention.
FIG. 5 is a view for describing that introduction of fine particles into a lower portion of a through-hole well causes the measurement intensity not to be affected by air bubbles in Example 2 of the present invention.
FIG. 6 is a view for describing that introduction of fine particles into a lower portion of a through-hole well reduces average reflected light intensity in a reflection image of the through-hole well in Example 2 of the present invention.
FIG. 7 is a view for describing that making a heat conduction plate in a lower portion of a measuring device transparent causes the measurement intensity not to be affected by air bubbles in Example 3 of the present invention.
FIG. 8 is a view for describing that forming a heat conduction plate in a lower portion of the measuring device in an uneven structure causes the measurement intensity not to be affected by air bubbles in Example 4 of the present invention.
FIG. 9 is a view for describing that coloring an oil in a measuring device in black causes the measurement intensity not to be affected by air bubbles in Example 5 of the present invention.
FIG. 10 is a view for describing that introduction of ink into a lower portion of a through-hole well causes the measurement intensity not to be affected by air bubbles in Example 6 of the present invention.
FIG. 11 is a view for describing a change in fluorescence intensity observed when a measuring device in which a reflection-suppressing mechanism is not introduced is heated at 85°C for a certain period of time in a comparative example.
FIG. 12 is a view for describing a change in fluorescence intensity observed when a measuring device in which a reflection-suppressing mechanism is introduced between a through-hole well and a heat conduction plate is heated at 85°C for a certain period of time in Example 7 of the present invention.
FIG. 13 is a flowchart of fluorescence intensity measurement with respect to temperature change in a measuring device in which a reflection-suppressing mechanism is introduced in Example 8 of the present invention.
FIG. 14 is a view for describing a change in fluorescence intensity with respect to a temperature change in a measuring device in which a reflection-suppressing mechanism is not introduced in a comparative example.
FIG. 15 is a view for describing a change in fluorescence intensity with respect to a temperature change in a measuring device in which a reflection-suppressing mechanism is introduced in Example 8 of the present invention.
FIG. 16 is a flowchart of fluorescence intensity measurement and reflection image measurement with respect to temperature change in a measuring device in which a reflection-suppressing mechanism is introduced in Example 9 of the present invention.
FIG. 17 is a view for describing a change in a coefficient of variation of reflected light intensity in a measuring device before and after introduction of a reflection-suppressing mechanism in Example 9 of the present invention.

### Description of Embodiments

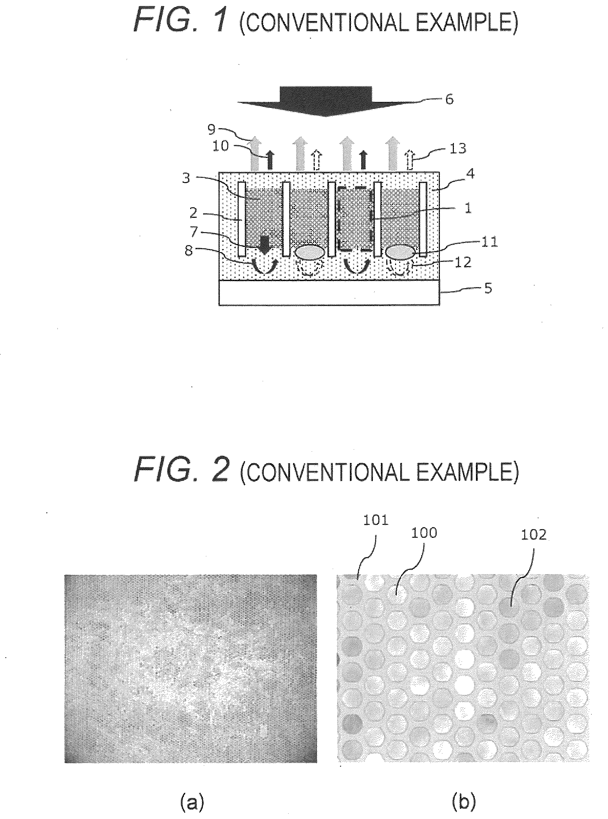

A conventional example will be described with reference to FIGS. 1 and 2.

FIG. 1 is a schematic view of a conventional measuring device holding a through-hole well 1 when fluorescence is measured. This measuring device includes a substrate 2 holding a plurality of through-hole wells 1, a mixed solution 3 of nucleic acid injected into the wells and a fluorescence-labeled probe, an oil 4 covering the through-hole wells 1, and a heat conduction plate 5.

In the fluorescence measurement using the measuring device, excitation light 6 is emitted to the through-hole wells 1. A part of the excitation light 6 becomes transmitted light 7 passing through the through-hole wells 1. The transmitted light 7 is reflected by the heat conduction plate 5 to become reflected light 8. The reflected light 8 passes through the through-hole wells 1 again. A reflected light intensity 10 is measured as the measurement light intensity of the wells together with a fluorescence intensity 9.

When an air bubble 11 is present in the lower portion of the through-hole well 1, the transmitted light 7 of the through-hole well 1 is diffused in the lower portion of the through-hole well 1 by the air bubble 11. Thus, light 12 that is not reflected by the heat conduction plate 5 is not detected as strong as the reflected light intensity 10. That is, when the air bubble is present, the measured light intensity is the sum of the fluorescence intensity 9 and the reflected light intensity 13 attenuated as compared with the original reflected light intensity 10.

It can be seen that unevenness thus exists in the measured light intensity of the well depending on the presence or absence of air bubbles. Therefore, in the measurement image, a portion where air bubbles are present is observed as dark measurement intensity.

FIG. 2 includes reflection images obtained by observing a substrate 101 holding a through-hole well 100 at a low magnification (FIG. 2(a)) and a high magnification (FIG. 2(b)). The reflection image of the through-hole well 100 is bright. Thus, the excitation light 6 passes through the through-hole well 1 and reflected by the heat conduction plate 5. When the through-hole well 100 and a through-hole well 102 are compared, there is a variation in brightness. This is due to in-plane variation in reflectance of the heat conduction plate 5.

It has been described above that the presence of the air bubble 11 affects the measurement in the conventional measurement chip. Hereinafter, it will be described that subjecting the heat conduction plate to colored surface treatment causes the air bubble 11 not to affect the measurement.

Hereinafter, embodiments of the present invention will be described. Description of parts common to the above-described conventional example may be omitted.

### Example 1

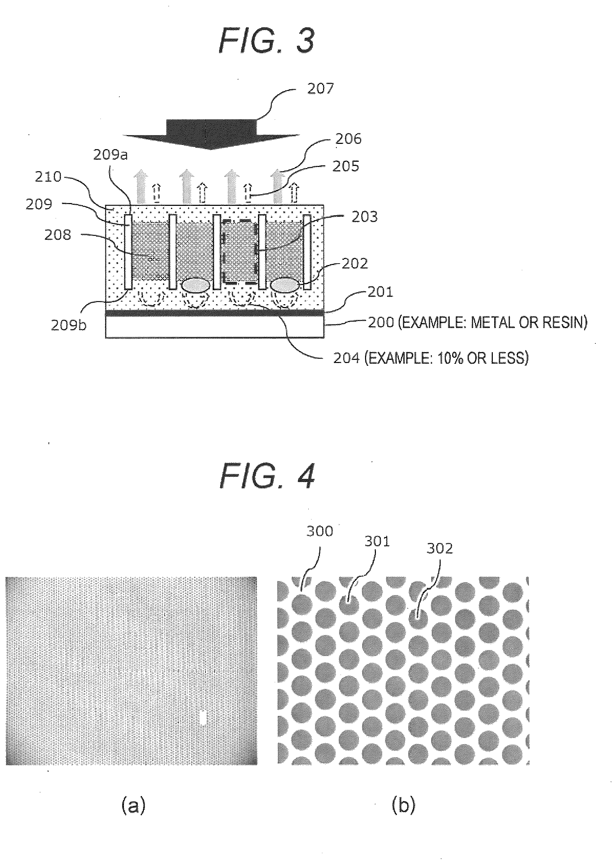

Hereinafter, Example 1 will be described with reference to FIGS. 3 and 4. In Example 1, the heat conduction plate is subjected to blackening treatment, which makes air bubbles difficult to be observed. Using the measuring device according to the present example makes it possible to measure the fluorescence intensity without the influence of air bubbles.

FIG. 3 is a schematic view illustrating that applying a black surface treatment film 201 to a heat conduction plate 200 at a lower portion of the measuring device makes an air bubble 202 observed in FIG. 1 difficult to be observed. The measuring device includes a substrate 209 having a plurality of through-hole wells 203 (through-holes) for introducing and dividing a mixed solution 208. The measuring device has an oil 210 covering a first surface 209a of the substrate 209 and a second surface 209b on the opposite side from the first surface of the substrate to close the through-hole wells 203.

The mixed solution 208 includes a nucleic acid solution. In the present specification and drawings, a reference to a mixed solution can be interpreted as a reference to a nucleic acid solution.

The measuring device includes a heat conduction plate 200 provided on the second surface 209b side to change the temperature of the substrate 209 (for example, to heat the substrate 209). It can be said that the second surface 209b is a surface of the substrate 209 on the heat conduction plate 200 side.

The material of the heat conduction plate 200 is, for example, metal or resin, or it may be glass. Using such a material makes it possible to configure a heat conduction plate suitable for the requirements of the measuring device. Other materials may also be used.

In the conventional configuration, there is a variation in reflected light intensity depending on the presence or absence of the air bubble 202 in the lower portion of the through-hole well 203. However, the measuring device according to the present example has a reflection-suppressing mechanism that suppresses reflection of excitation light 207 emitted from the first surface 209a side (for example, reflection to the first surface 209a side) . In the present example, the reflection suppressing mechanism is a colored surface treatment structure of the heat conduction plate 200.

As a specific example, the reflection-suppressing mechanism includes a black surface treatment film 201 as a reflection-suppressing film. The surface treatment film 201 is disposed on the heat conduction plate 200. The surface treatment film 201 absorbs excitation light (or light having the same wavelength as the excitation light). Applying the black surface treatment film 201 to the heat conduction plate 200 causes reflected light 204 from the heat conduction plate 200 to generate attenuated reflected light intensity 205. Thus, the measured light intensity of each through-hole well 203 is the sum of the fluorescence intensity 206 and the attenuated reflected light intensity 205. Therefore, the air bubbles are prevented from being observed as unevenness on the measurement image.

The structure and forming method of the surface treatment film 201 can be appropriately designed by those skilled in the art. The thickness of the surface treatment film 201 varies depending on the surface treatment step. As some examples, an anodized film such as a film formed through an alumite treatment has a thickness of about 5 to 40 µm, and a film formed through electroplating has a thickness of about 2 to 20 µm. The thickness of a film formed through spray coating is about 15 to 30 µm. In the case of the present example, the film thickness is desirably 40 µm or less.

FIG. 4 includes reflection images obtained by observing a substrate 300 after applying the black surface treatment film 201 to the heat conduction plate at low magnification (FIG. 4(a)) and high magnification (FIG. 4(b)). The reflection image in a through-hole well 301 is dark. In the comparison of the reflected light intensity between the through-hole well 301 and a through-hole well 302, it can be seen that there is no variation in the reflected light intensity. It can also be seen that the reflected light intensity is small as a whole. From this reflection image, it can be said that reflection from the heat conduction plate 200 is suppressed.

In the present example, the black surface treatment film 201 is used as the colored surface treatment structure. In order to suppress reflection from the heat conduction plate 200, the reflectance thereof is important. In the present example, "colored" means that the reflectance is 10% or less over a wavelength of 400 nm to 700 nm, for example, and means black as a specific example. With such a colored surface treatment structure, reflection from the heat conduction plate 200 is suppressed.

As described above, the measuring device according to the present example can suppress the unevenness of the fluorescence image and the fluctuation in the fluorescence intensity caused by the influence of air bubbles in the melting curve analysis of the target gene.

### Example 2

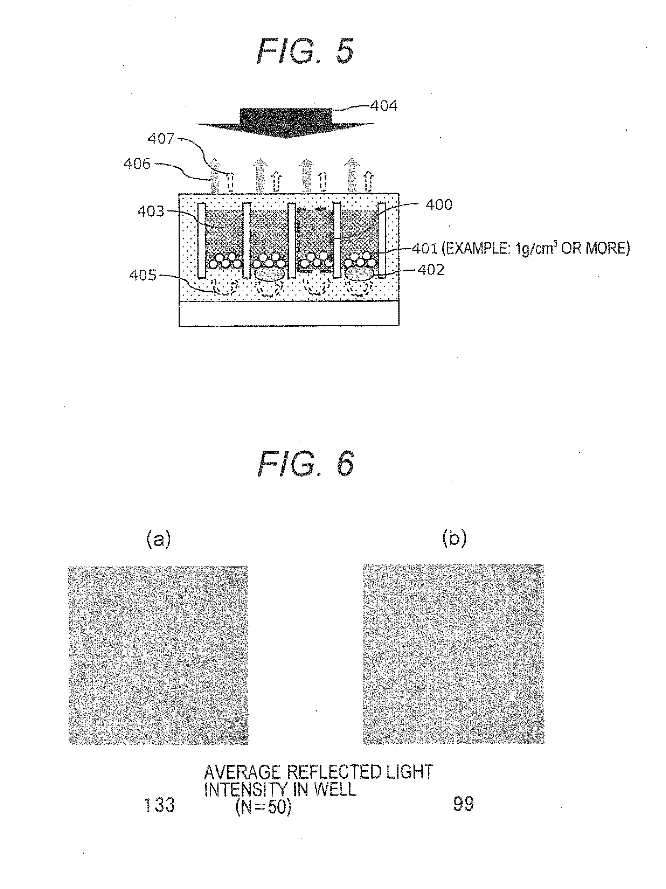

Hereinafter, Example 2 will be described with reference to FIGS. 5 and 6. In Example 2, fine particles 401 are injected into the lower portion of a through-hole well 400, which makes an air bubble 402 difficult to be observed. Using the measuring device according to the present example makes it possible to measure the fluorescence intensity without the influence of the air bubble 402. Hereinafter, description of parts common to Example 1 may be omitted.

FIG. 5 is a schematic view illustrating a state where fluorescence measurement is performed in a case where fine particles 401 are introduced into a lower portion of a mixed solution 403 of a nucleic acid and a fluorescence-labeled probe injected into the through-hole well 400 in the measuring device. The reflection-suppressing mechanism according to the present example includes the fine particles 401 introduced into the through-hole well 400. It is preferable that the fine particles 401 do not allow excitation light 404 to pass therethrough.

When the fine particles 401 are not present, a phenomenon similar to that in FIG. 1 occurs. However, since the excitation light 404 does not pass through the well because of the presence of the fine particles 401, reflected light 405 is suppressed by the heat conduction plate. Thus, the measured light intensity is the sum of a fluorescence intensity 406 and an attenuated reflected light intensity 407. This makes the air bubble 402 difficult to be observed in the measurement image.

FIG. 6 illustrates a comparison between reflection images of the measuring devices illustrated in FIGS. 1 and 5. FIG. 6(a) is a reflection image obtained by observing a conventional measuring device. FIG. 6(b) is a reflection image obtained by observing a measuring device according to the present example into which fine particles are injected.

In the example of FIG. 6(a), the average reflected light intensity averaged for 50 through-hole wells was 133 (any unit). In the example of FIG. 6(b), the average reflected light intensity was 99. When the average reflected light intensity of the plurality of through-hole wells is focused, the average reflected light intensity of the plurality of wells is decreased by injecting fine particles into the through-hole wells. Thus, excitation light is prevented from passing through the through-hole well and being reflected on the heat conduction plate.

Conditions of the fine particles to be injected include size, specific gravity, surface treatment, and number. The size (for example, diameter) is desirably 30 nm or more. When the fine particles scatter light in the visible range to the near-infrared range, the scattering phenomenon is based on Mie scattering theory. Since Mie scattering shows significant scattering intensity from about 30 nm, this value can be set as a lower limit value. On the other hand, the size of the fine particles is preferably smaller than that of the through-hole well into which the fine particles are injected. Thus, when the well size is 60 µm, the upper limit value of the fine particles can be 60 µm.

As a condition determined from the viewpoint of specific gravity, it is preferable that the fine particles settle at the bottom of the through-hole well. Thus, it is preferable to increase the specific gravity of the fine particles with respect to the mixed solution of the nucleic acid and the fluorescence-labeled probe to be injected into the through-hole well. That is, it is preferable that the fine particles have a specific gravity larger than that of the mixed solution.

In one example, fine particles having a specific gravity larger than 1, such as polymer fine particles (polystyrene, specific gravity: 1.04 to 1.07 g/cm³), magnetic fine particles (iron, specific gravity: 7.85 g/cm³), and metal fine particles (silver, specific gravity: 10.49 g/cm³), are used as the fine particles (that is, the specific gravity of the fine particles is 1 g/cm³ or more). In such a case, the fine particles settle in the through-hole well.

As conditions required from the viewpoint of surface treatment of fine particles, it is beneficial to examine the affinity with the mixed solution of a nucleic acid and a fluorescence-labeled probe. Since the mixed solution of a nucleic acid and a fluorescence-labeled probe is a watersoluble solution, it is suitable that the fine particles are hydrophilically treated (for example, hydrophilically coated). To prevent the nucleic acid from being adsorbed to the fine particle, it is preferable to negatively charge the surfaces of the fine particles.

In one example, a treatment of disposing a carboxy group on the surfaces of the fine particles is performed so that the fine particle exhibits hydrophilicity. In another example, it is possible to set a magnet in the lower portion of the measuring device and cause the hydrophilicized magnetic fine particles to settle in the lower portion of the through-hole well by using magnetic force. Since the periphery of the through-hole well is oil, the hydrophilic magnetic fine particles are retained in the lower portion of the through-hole well. Thus, using this method makes it possible to control the position of the magnetic fine particles.

As a condition determined from the viewpoint of the number, a condition that the fine particle sectional area × the number of injections is equal to or less than the bottom area of the through-hole well: (fine particle sectional area) × (number of fine particles to be injected) ≤ (through-hole well bottom area) is preferably satisfied. It is not always necessary to cover the entire bottom area of the through-hole well with fine particles. In one example, it has been confirmed that the influence of air bubbles is reduced using the condition that about 10% of the bottom area of the well is covered with fine particles.

As described above, the measuring device according to the present example can suppress the unevenness of the fluorescence image and the fluctuation in the fluorescence intensity caused by the influence of air bubbles in the melting curve analysis of the target gene.

### Example 3

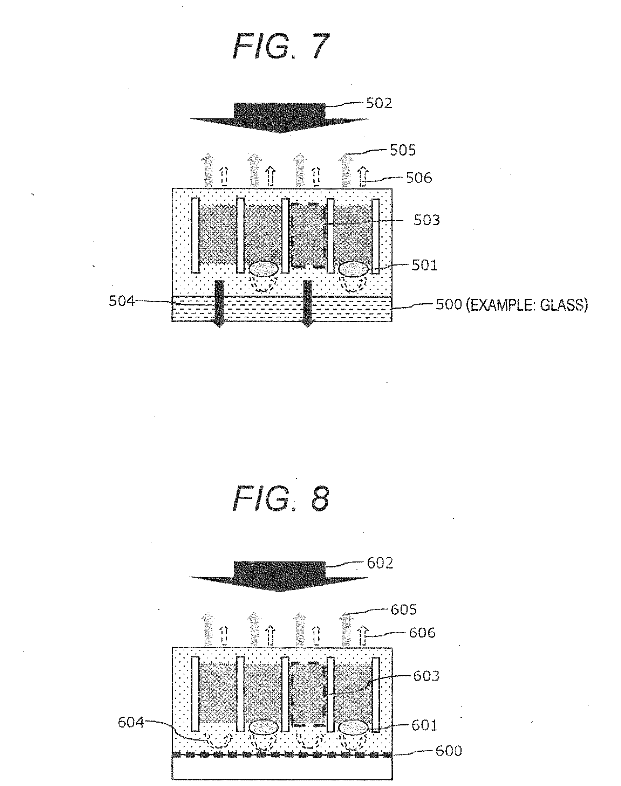

Hereinafter, Example 3 will be described with reference to FIG. 7. In Example 3, as the reflection-suppressing mechanism, the lower portion of the measuring device is formed into a transparent heat conduction plate 500, which makes an air bubble 501 difficult to be observed. Using the measuring device according to the present example makes it possible to measure the fluorescence intensity without the influence of the air bubble 501. Hereinafter, description of parts common to Examples 1 and 2 may be omitted.

FIG. 7 is a schematic view illustrating a state where fluorescence is measured in a case where the lower portion of the measuring device is formed into the transparent heat conduction plate 500. Excitation light 502 passes through a through-hole well 503 and reaches the transparent heat conduction plate 500. At this time, the excitation light 502 becomes light 504 passing through the heat conduction plate 500 because of the transparent heat conduction plate. This makes it possible to suppress reflection of excitation light.

Thus, the measured light intensity is the sum of a fluorescence intensity 505 and an attenuated reflected light intensity 506. This makes the air bubble 501 difficult to be observed in the measurement image.

Examples of the material of the transparent heat conduction plate 500 include plastic such as polycarbonate and glass. The transparent heat conduction plate 500 may be a transparent conductive substrate obtained by doping a glass substrate with indium tin oxide or the like.

As described above, the measuring device according to the present example can suppress the unevenness of the fluorescence image and the fluctuation in the fluorescence intensity caused by the influence of air bubbles in the melting curve analysis of the target gene.

### Example 4

Hereinafter, Example 4 will be described with reference to FIG. 8. In Example 4, a heat conduction plate 600 having an uneven structure is used, which makes an air bubble 601 difficult to be observed. Using the measuring device according to the present example makes it possible to measure the fluorescence intensity without the influence of the air bubble 601. Hereinafter, description of parts common to Examples 1 to 3 may be omitted.

FIG. 8 is a schematic view illustrating a state where fluorescence is measured in a case where the heat conduction plate 600 having an uneven structure is used as the lower portion of the measuring device. In this manner, the reflection-suppressing mechanism according to the present example includes an uneven structure, and the uneven structure is disposed on the heat conduction plate 600 having an uneven structure.

Excitation light 602 passes through a through-hole well 603 and reaches the heat conduction plate 600 having an uneven structure. At this time, reflected light 604 of the excitation light can be suppressed by the uneven structure. Thus, the measured light intensity is the sum of a fluorescence intensity 605 and an attenuated reflected light intensity 606. This makes the air bubble 601 difficult to be observed in the measurement image. As a condition required from the viewpoint of reducing reflection due to unevenness, it is preferable that the structure is periodic. It is more preferable that the pitch (spatial repetition period) of the uneven structure is 1 µm or less.

As described above, the measuring device according to the present example can suppress the unevenness of the fluorescence image and the fluctuation in the fluorescence intensity caused by the influence of air bubbles in the melting curve analysis of the target gene.

### Example 5

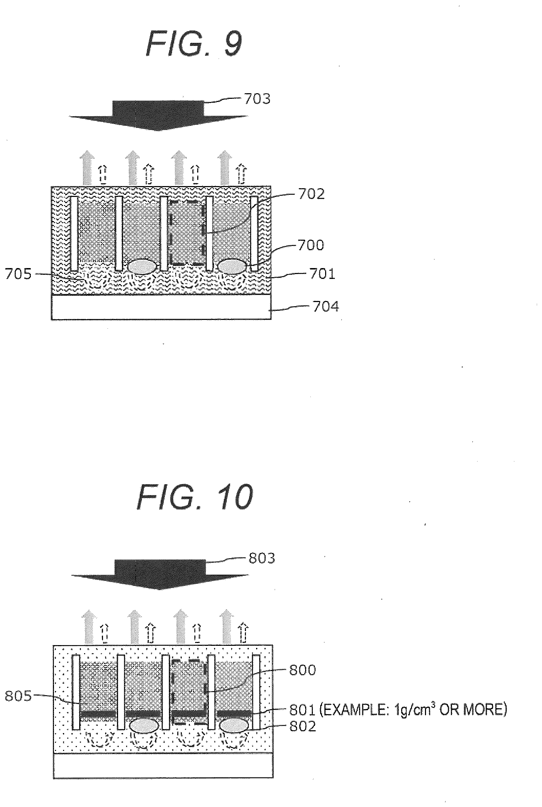

Hereinafter, Example 5 will be described with reference to FIG. 9. In Example 5, the oil covering the through-holes is colored, which makes an air bubble 700 difficult to be observed. Using the measuring device according to the present example makes it possible to measure the fluorescence intensity without the influence of the air bubble 700. Hereinafter, description of parts common to Examples 1 to 4 may be omitted.

FIG. 9 is a schematic view illustrating a state where fluorescence is measured in a case where a through-hole well 702 is covered with a colored oil 701 in the measuring device. In the present example, the oil 701 is colored in black. The colored oil 701 is not necessarily limited to those subjected to the coloring treatment, and the color is not limited to black.

Excitation light 703 is absorbed by the colored oil 701. Thus, reflected light 705 from a heat conduction plate 704 can be suppressed. This makes the air bubble 700 difficult to be observed in the measurement image. The reflectance of the coloring pigment is important in the coloring of the oil. In the present example, "colored" means that the reflectance is 10% or less over a wavelength of 400 nm to 700 nm, for example. With such a reflectance, reflection of the excitation light can be efficiently suppressed.

The oil 701 may actually be present on the upper side of the through-hole well 702, and in such a case, fluorescence may also be shielded. Here, it is assumed that the reflectance of the excitation light and the fluorescence in the oil 701 is 10%, and the transmittance is 10%. Of the total amount of the excitation light assumed to be 100%, 10% is incident on the through-hole well 702, 1% is reflected at the lower end of the through-hole well 702, and 0.1% is transmitted to the upper side of the through-hole well 702 and detected. On the other hand, of the total amount of the excitation light assumed to be 100%, 10% is transmitted to the upper side of the through-hole well 702 and detected. In this manner, it can be said that the excitation light is negligible with respect to the fluorescence on the upper side of the through-hole well 702, and the reflection of the excitation light is efficiently suppressed.

As described above, the measuring device according to the present example can suppress the unevenness of the fluorescence image and the fluctuation in the fluorescence intensity caused by the influence of air bubbles in the melting curve analysis of the target gene.

### Example 6

Hereinafter, Example 6 will be described with reference to FIG. 10. In Example 6, an ink 801 is introduced into the lower portion of a through-hole well 800, which makes an air bubble 802 difficult to be observed. Using the measuring device according to the present example makes it possible to measure the fluorescence intensity without the influence of the air bubble 802. Hereinafter, description of parts common to Examples 1 to 5 may be omitted.

FIG. 10 is a schematic view illustrating a state where fluorescence is measured in a case where the ink 801 is injected into the lower portion of the through-hole well 800. In the present example, the reflection-suppressing mechanism includes the ink 801 introduced into the through-hole well 800. After passing through the through-hole well 800, excitation light 803 is absorbed by the ink 801. This makes the air bubble 802 difficult to be observed in the measurement image.

In the present example, the ink 801 is desirably a pigment ink. In the pigment ink, the specific gravity is preferably larger than that of the mixed solution of a nucleic acid and a fluorescence-labeled probe as the condition determined from the viewpoint of the specific gravity. That is, it is preferable that the ink 801 has a specific gravity larger than that of a mixed solution 805. Satisfying this condition makes it possible to cause the ink to settle to the lower portion of the through-hole well. As a specific example, the specific gravity of the ink 801 is preferably 1 g/cm³ or more. As the pigment ink, carbon black (specific gravity: 1.7 to 1.8 g/cm³) or the like may be used.

As described above, the measuring device according to the present example can suppress the unevenness of the fluorescence image and the fluctuation in the fluorescence intensity caused by the influence of air bubbles in the melting curve analysis of the target gene.

### Example 7

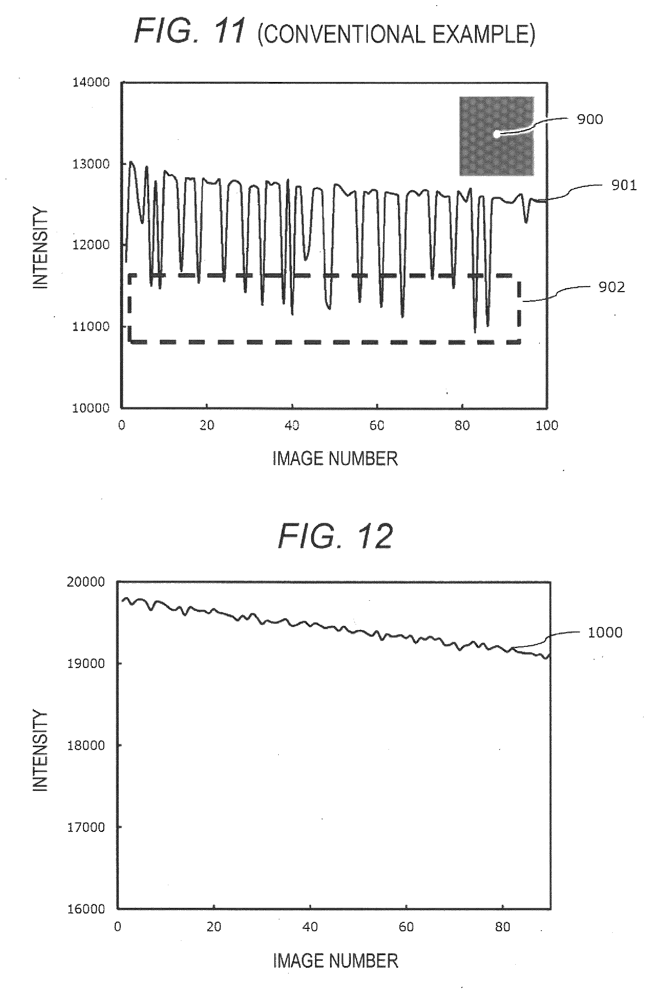

Hereinafter, Example 7 will be described with reference to FIGS. 11 and 12. Example 7 shows an effect that air bubbles are not actually observed by using the measuring device according to Example 1.

In this measurement, the measuring device is heated for a certain period of time so as to be maintained at 85°C. The measuring device is irradiated with excitation light in the visible range to acquire a fluorescence image. The exposure time at the time of fluorescence image acquisition is 1300 ms. 80 or more fluorescence images are continuously photographed to evaluate the presence or absence of the influence of air bubbles.

FIG. 11 illustrates a fluorescence intensity change 901 of a single well 900 in the measuring device illustrated in FIG. 1 not provided with the reflection-suppressing mechanism as a comparative example. A part of the fluorescence image of the through-hole well is illustrated in the upper right of the graph (only the single well 900 is modified white for visibility). In the graph, the horizontal axis represents the number of fluorescence images, and the vertical axis represents the fluorescence intensity. The image number represents the order in which the images were acquired, and thus, the horizontal axis corresponds to time.

The data in the graph is the fluorescence intensity change of the single well 900 in each fluorescence image. In the graph, the fluorescence intensity does not always show a constant value, and a steep fluorescence intensity change 902 occurs. When this steep fluorescence intensity change 902 occurs, air bubbles are present in the lower portion of the well. Thus, the presence of air bubbles can be confirmed from the steep fluorescence intensity change 902 of the fluorescence intensity.

FIG. 12 illustrates a fluorescence intensity change 1000 of a single well in the measuring device according to Example 1 to which the reflection-suppressing mechanism is applied. It can be seen that a steep change in fluorescence intensity is not observed as compared with the case of FIG. 11. Thus, it is possible to suppress the influence of air bubbles by using Example 1. It is considered that the same effect is obtained from the measuring device according to Examples 2 to 6.

As described above, the measuring device according to the present example can suppress the unevenness of the fluorescence image and the fluctuation in the fluorescence intensity caused by the influence of air bubbles in the melting curve analysis of the target gene.

### Example 8

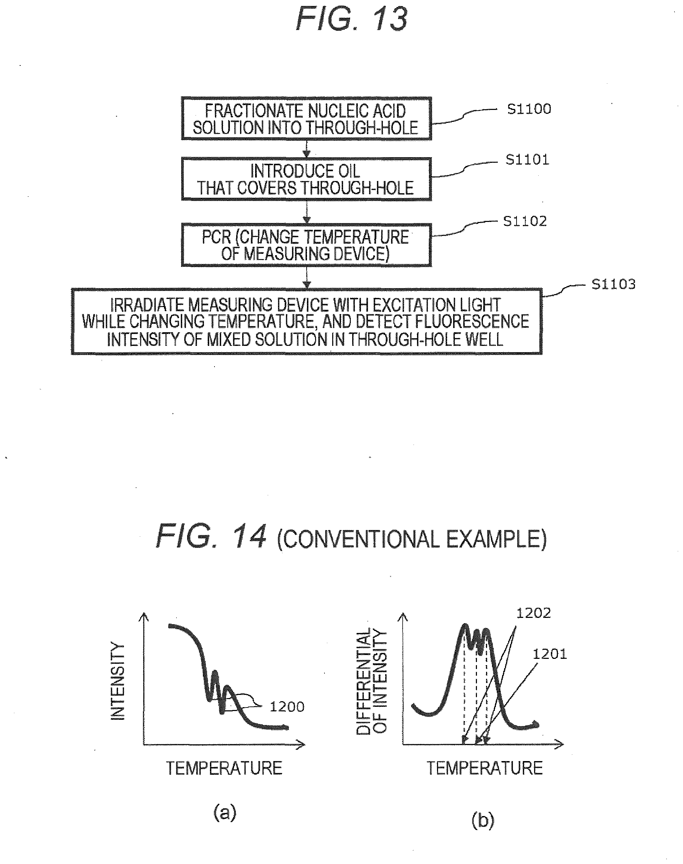

Hereinafter, Example 8 will be described with reference to FIGS. 13 to 15. Example 8 illustrates a measuring method for melting curve analysis using the measuring device of Examples 1 to 6. Using the measuring device according to the present example makes it possible to measure melting curve analysis with high accuracy.

FIG. 13 illustrates a measurement flow from injecting a mixed solution of a nucleic acid and a fluorescence-labeled probe into the measuring device according to the present example to performing melting curve analysis.

First, a mixed solution of a nucleic acid and a fluorescence-labeled probe is introduced into the measuring device and fractionated into each of the through-hole wells to fractionate the nucleic acid into the through-hole wells (S1100).

Next, an oil is introduced in such a manner as to cover the periphery of the through-hole well (S1101). In the example of FIG. 3, the oil is introduced to cover the first surface 209a of the substrate 209 and the second surface 209b on the opposite side from the first surface 209a of the substrate 209 to close the through-hole well 203 into which the mixed solution 208 has been introduced.

After the oil is introduced, PCR is performed to amplify the nucleic acid in the through-hole well (S1102). In the example of FIG. 3, the temperature of the measuring device (in particular, the temperature of the substrate 209) is changed by the heat conduction plate 200.

Finally, the measurement device is irradiated with excitation light while the temperature is changed to detect the fluorescence intensity from the mixed solution of the nucleic acid and the fluorescence-labeled probe in the through-hole well (S1103). In the example of FIG. 3, the excitation light 207 is emitted from the first surface 209a side of the substrate 209, and fluorescence is detected from the first surface 209a side, whereby the mixed solution 208 is measured.

FIG. 14 illustrates an example of a melting curve analysis result in a conventional measuring device not provided with the reflection-suppressing mechanism as a comparative example. FIG. 14(a) is a graph in which the fluorescence intensity change with respect to temperature change in a single well in the measuring device is plotted. A decrease in fluorescence intensity can be confirmed. When air bubbles are observed during this decrease, a steep fluorescence intensity decrease 1200 is observed.

FIG. 14(b) is obtained by differentiating the temperature with respect to the fluorescence intensity change of FIG. 14(a), and a differential curve of the melting curve is calculated. The melting temperature is calculated from the peak of the differential curve. In the differential curve where air bubbles were observed, not only a true melting temperature 1201 but also a melting temperature artifact 1202 is observed. This artifact is caused from a steep fluorescence intensity decrease 1200. Thus, the influence of air bubbles greatly affects the melting curve analysis.

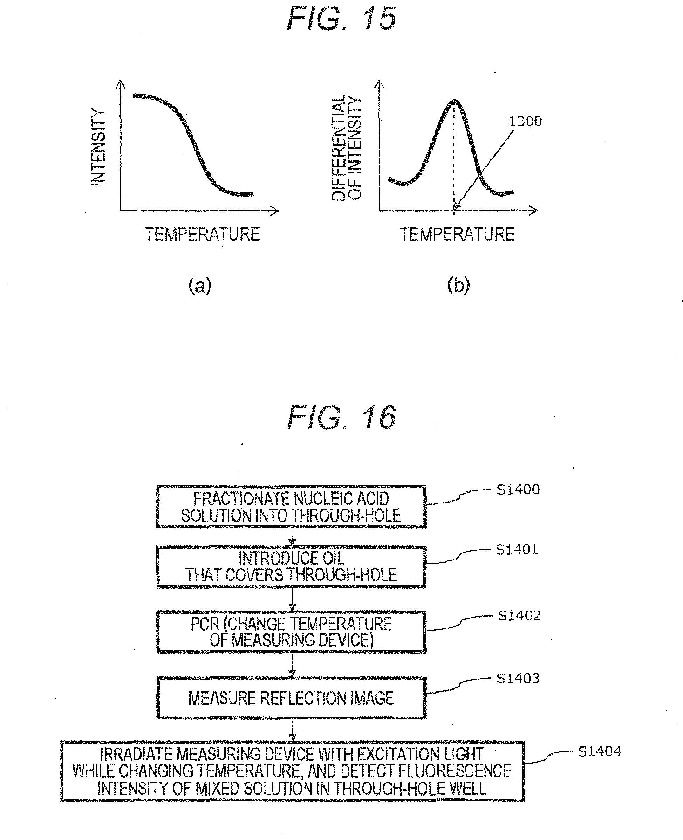

FIG. 15 illustrates an example of a melting curve analysis result in the present example. In FIG. 15(a), the fluorescence intensity change with respect to the temperature change in a single well in the measuring device is plotted. A monotonic decrease in fluorescence intensity can be confirmed.

FIG. 15(b) is obtained by differentiating the temperature with respect to the fluorescence intensity change of FIG. 15(a). In the measuring device of the present example, air bubbles are hardly observed. Thereby, a steep decrease in fluorescence intensity or the like is hardly observed in the melting curve. Thus, only a true melting temperature 1300 is observed in the differential curve of the melting curve. Therefore, the measuring device according to the present example can suppress unnecessary artifacts, and highly accurate melting curve analysis can be performed.

As described above, the measuring device and the measuring method according to the present example can suppress the unevenness of the fluorescence image and the fluctuation in the fluorescence intensity caused by the influence of air bubbles in the melting curve analysis of the target gene.

### Example 9

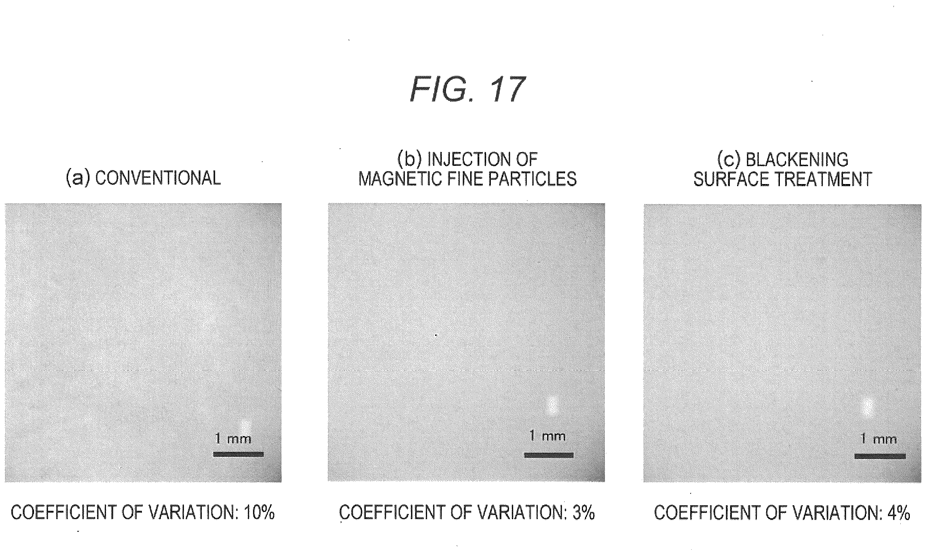

Hereinafter, Example 9 will be described with reference to FIGS. 16 and 17. Example 9 illustrates a method for confirming that the effect of making air bubbles difficult to be observed is obtained by blackening surface treatment on the heat conduction plate of Example 1 or injection of fine particles into through-hole wells of Example 2.

FIG. 16 illustrates a measurement flow in which the operation of reflection image measurement is added to the measurement flow diagram of Example 8 illustrated in FIG. 13. Steps S1400 to S1402 and S1404 in FIG. 16 can be the same as the steps S1100 to S1102 and S1103 in FIG. 13, respectively.

After step S1402, the reflection image of the measuring device is measured (S1403). In the example of FIG. 3, the reflection image is measured by irradiating excitation light 207 or white light from the first surface 209a side of the substrate 209.

The above description is merely an example, in which the operation of the reflection image measurement is performed after PCR (S1402). Alternatively, the operation may be performed after an oil introduction step (S1401) of covering the through-hole. The step of S1403 can be performed after step S1401 and before step S1404 ends.

FIG. 17 illustrates a reflection image actually observed in each measuring device. As the reflection image, an image focused on the heat conduction plate is acquired.

In the case of the conventional measuring device (FIG. 17(a)) not subjected to processing such as the reflection suppressing mechanism, unevenness of reflected light is observed. The coefficient of variation of the reflected light intensity is 10%. Here, the coefficient of variation is a value obtained by dividing the standard deviation of the reflected light intensity by the average of the reflected light intensities for a plurality of through-hole wells.

On the other hand, when the reflection-suppressing mechanism such as injection of magnetic fine particles (Example 2, FIG. 17(b)) or blackening surface treatment on the heat conduction plate (Example 1, FIG. 17(c)) is performed, the coefficients of variation of the reflected light intensity are 3% and 4%, respectively.

These values are merely examples. However, as compared with the conventional measuring device, the coefficient of variation of the reflected light intensity decreases in the measuring device according to each example of the present invention. Thus, the performance of the measuring device according to the present example can be evaluated by evaluating the reflection image before performing the melting curve analysis.

As described above, the measuring device and the measuring method according to the present example can suppress the unevenness of the fluorescence image and the fluctuation in the fluorescence intensity caused by the influence of air bubbles in the melting curve analysis of the target gene.

The present invention is not limited to the above-described examples but includes various modifications. For example, the above-described examples have been described in detail for easy understanding of the present invention, and the present invention is not necessarily limited to those having all the described configurations. A part of the configuration of a certain example can be replaced with the configuration of another example, and the configuration of a certain example can be added to the configuration of another example. A part of a configuration of each example can be added to, deleted from, and replaced with a part of another configuration.

### Reference Signs List

200 heat conduction plate
201 surface treatment film (reflection-suppressing mechanism, reflection-suppressing film)
202 air bubbles
203 -through-hole well (through-hole)
204 reflected light
205 attenuated reflected light intensity
206 fluorescence intensity
207 excitation light
208 mixed solution (nucleic acid solution)
209 substrate
209a first surface
209b second surface
210 oil
300 substrate
301 through-hole well (through-hole)
302 through-hole well (through-hole)
400 through-hole well (through-hole)
401 fine particles (reflection-suppressing mechanism)
402 air bubbles
403 mixed solution (nucleic acid solution)
404 excitation light
405 reflected light
406 fluorescence intensity
407 attenuated reflected light intensity
500 transparent heat conduction plate (reflection-suppressing mechanism)
501 air bubbles
502 excitation light
503 through-hole well (through-hole)
504 light passing through heat conduction plate
505 fluorescence intensity
506 attenuated reflected light intensity
600 heat conduction plate with uneven structure
(reflection-suppressing mechanism)
601 air bubbles
602 excitation light
603 through-hole well (through-hole)
604 reflected light
605 fluorescence intensity
606 attenuated reflected light intensity
700 air bubbles
701 colored oil (reflection-suppressing mechanism)
702 through-hole well (through-hole)
703 excitation light
704 heat conduction plate
705 reflected light
800 through-hole well (through-hole)
801 ink (reflection-suppressing mechanism)
802 air bubbles
803 excitation light
805 mixed solution (nucleic acid solution)
900 single well (through-hole)
901 fluorescence intensity change
902 steep fluorescence intensity change
1000 fluorescence intensity change
1200 steep fluorescence intensity decrease
1201 true melting temperature
1202 melting temperature artifact
1300 true melting temperature

## Claims

1. A measuring device comprising:
a substrate including a plurality of through-holes for introducing and dividing a nucleic acid solution;
an oil covering a first surface of the substrate and a second surface on an opposite side from the first surface of the substrate to close the through-hole;
a heat conduction plate provided on the second surface side for heating the substrate; and
a reflection-suppressing mechanism configured to suppress reflection of excitation light emitted from the first surface side.

2. The measuring device according to claim 1, wherein
the reflection-suppressing mechanism includes fine particles introduced into the through-hole,
the fine particles are hydrophilically coated, and
the fine particles have a specific gravity larger than a specific gravity of the nucleic acid solution.

3. The measuring device according to claim 2, wherein the specific gravity of the fine particles is 1 g/cm³ or more.

4. The measuring device according to claim 1, wherein
the reflection-suppressing mechanism includes an ink introduced into the through-holes, and
the ink has a specific gravity larger than a specific gravity of the nucleic acid solution.

5. The measuring device according to claim 4, wherein the specific gravity of the ink is 1 g/cm³ or more.

6. The measuring device according to claim 1, wherein
the reflection-suppressing mechanism includes a reflection-suppressing film,
the reflection-suppressing film is disposed on the heat conduction plate, and
the reflection-suppressing film absorbs the excitation light.

7. The measuring device according to claim 1, wherein
the reflection-suppressing mechanism includes an uneven structure,
the uneven structure is disposed on the heat conduction plate, and
the uneven structure has a pitch of 1 µm or less.

8. The measuring device according to claim 1, wherein the reflection-suppressing mechanism is a colored surface treatment structure of the heat conduction plate.

9. The measuring device according to claim 1, wherein a material of the heat conduction plate is any of metal, resin, or glass.

10. The measuring device according to claim 1, wherein the oil is colored.

11. The measuring device according to claim 8 or 10, wherein the colored means having a reflectance of 10% or less over a wavelength of 400 nm to 700 nm.

12. A measuring method using a measuring device, the measuring device comprising:
a substrate including a plurality of through-holes;
a heat conduction plate provided to change a temperature of the substrate; and
a reflection-suppressing mechanism configured to suppress reflection of excitation light emitted from a first surface side of the substrate on an opposite side from the heat conduction plate side,
the measuring method comprising:
a step of introducing a nucleic acid solution into the substrate and fractionating the nucleic acid solution;
a step of introducing an oil that covers a first surface of the substrate and a second surface on an opposite side from the first surface of the substrate to close the through-holes into which the nucleic acid solution is introduced;
a step of changing the temperature of the substrate with the heat conduction plate; and
a step of emitting excitation light from the first surface side of the substrate while changing the temperature, detecting fluorescence from the first surface side, and measuring the nucleic acid solution.

13. The measuring method according to claim 12, wherein
the reflection-suppressing mechanism includes fine particles,
the fine particles are hydrophilically coated, and
the fine particles have a specific gravity larger than a specific gravity of the nucleic acid solution.

14. The measuring method according to claim 13, wherein the specific gravity of the fine particles is 1 g/cm³ or more.

15. The measuring method according to claim 12, wherein
the reflection-suppressing mechanism includes an ink introduced into the through-holes, and
the ink has a specific gravity larger than a specific gravity of the nucleic acid solution.

16. The measuring method according to claim 15, wherein the specific gravity of the ink is 1 g/cm³ or more.

17. The measuring method according to any one of claims 13 to 16, the method further comprising a step of measuring a reflection image by irradiating the substrate with the excitation light or white light from the first surface side after the step of introducing the oil and before the step of measuring the nucleic acid solution is completed.

18. The measuring method according to claim 12, wherein
the reflection-suppressing mechanism includes a reflection-suppressing film,
the reflection-suppressing film is disposed on the heat conduction plate, and
the reflection-suppressing film absorbs the excitation light.

19. The measuring method according to claim 12, wherein
the reflection-suppressing mechanism includes an uneven structure,
the uneven structure is disposed on the heat conduction plate, and
the uneven structure has a pitch of 1 µm or less.

20. The measuring method according to claim 12, wherein the reflection-suppressing mechanism is a colored surface treatment structure of the heat conduction plate.

21. The measuring method according to claim 12, wherein a material of the heat conduction plate is any of metal, resin, or glass.

22. The measuring method according to claim 12, wherein the oil is colored.

23. The measuring method according to claim 20 or 22, wherein the colored means having a reflectance of 10% or less over a wavelength of 400 nm to 700 nm.

24. The measuring method according to any one of claims 18 to 22, the method further comprising a step of measuring a reflection image by irradiating the substrate with excitation light or white light from the first surface side after the step of introducing the oil and before the step of measuring the nucleic acid solution is completed.
